# EUROPEAN PATENT SPECIFICATION

(11) **EP 2 967 452 B1**
(45) Date of publication and mention of the grant of the patent: **04.12.2019**
(21) Application number: 14765236.6
(22) Date of filing: 14.03.2014
(51) Int. Cl.: A61B 5/145

(54) **MEDICAL DEVICE DATA PROCESSING AND COMMUNICATION METHODS AND SYSTEMS**
DATENVERARBEITUNG EINER MEDIZINISCHEN VORRICHTUNG UND KOMMUNIKATIONSVERFAHREN UND -SYSTEME
PROCÉDÉS ET SYSTÈMES DE COMMUNICATION ET DE TRAITEMENT DE DONNÉES DE DISPOSITIF MÉDICAL

(30) Priority: 15.03.2013 US 201361801759 P
(43) Date of publication of application: 20.01.2016
(73) Proprietor: Abbott Diabetes Care Inc., Alameda, CA 94502 (US)
(72) Inventor: TAUB, Marc, Barry, Mountain View, CA 94040 (US); BUDIMAN, Erwin, Satrya, Fremont, CA 94555 (US); HUA, Xuandong, Mountain View, CA 94040 (US); LOVE, Michael, Pleasanton, CA 94566 (US)
(74) Representative: Booth, Catherine Louise
(86) International application number: PCT/US2014/029635
(87) International publication number: WO 2014/145001

(56) References cited:
- US-A1- 2009 006 034
- US-A1- 2009 006 034
- US-A1- 2009 177 068
- US-A1- 2010 198 142
- US-A1- 2011 289 497

## Description

### BACKGROUND

The detection and/or monitoring of glucose levels or other analytes, such as lactate, oxygen, AIC, or the like, in certain individuals is vitally important to their health. For example, the monitoring of glucose is particularly important to individuals with diabetes. Diabetics generally monitor glucose levels to determine if their glucose levels are being maintained within a clinically safe range, and may also use this information to determine if and/or when insulin is needed to reduce glucose levels in their bodies or when additional glucose is needed to raise the level of glucose in their bodies.

Growing clinical data demonstrates a strong correlation between the frequency of glucose monitoring and glycemic control. Despite such correlation, many individuals diagnosed with a diabetic condition do not monitor their glucose levels as frequently as they should due to a combination of factors including convenience, testing discretion, pain associated with glucose testing, and cost.

Devices have been developed for the automatic monitoring of analyte(s), such as glucose, in bodily fluid such as in the blood stream or in interstitial fluid ("ISF"), or other biological fluid. Some of these analyte measuring devices are configured so that at least a portion of the devices are positioned below a skin surface of a user, e.g., in a blood vessel or in the subcutaneous tissue of a user, so that the monitoring is accomplished in vivo.

With the continued development of analyte monitoring devices and systems, there is a need for such analyte monitoring devices, systems, and methods, as well as for processes for manufacturing analyte monitoring devices and systems that are cost effective, convenient, and with reduced pain, provide discreet monitoring to encourage frequent analyte monitoring to improve glycemic control. US-A-2009/0006034 discloses a method and apparatus for providing data processing and control in a medical communication system. Claim 1 is characterised over this disclosure.

In accordance with the present invention, there is provided an apparatus as claimed in claim 1 and a method as claimed in claim 12. Embodiments of the subject disclosure include in vivo analyte monitoring devices, systems, kits, and processes of analyte monitoring and making analyte monitoring devices, systems and kits. Included are on-body (i.e., at least a portion of a device, system or a component thereof is maintained on the body of a user to monitor an analyte), physiological monitoring devices configured for real time measurement/monitoring of desired analyte level such as a glucose level over one or more predetermined time periods such as one or more predetermined monitoring time periods. Embodiments include transcutaneously positioned analyte sensors that are electrically coupled with electronics provided in a housing that is designed to be attached to the body of a user, for example, to a skin surface of a user, during the usage life of the analyte sensors or predetermined monitoring time periods. For example, on body electronics assembly include electronics that are operatively coupled to an analyte sensor and provided in a housing for placement on the body of a user.

Such device and system with analyte sensors provide continuous or periodic analyte level monitoring that is executed automatically, or semi-automatically by control logic or routines programmed or programmable in the monitoring devices or systems. As used herein, continuous, automatic, and/or periodic monitoring refer to the in vivo monitoring or detection of analyte levels with transcutaneously positioned analyte sensors.

In certain embodiments, the results of the in vivo monitored analyte level are automatically communicated from an electronics unit to another device or component of the system. That is, when the results are available, the results are automatically transmitted to a display device (or other user interaction device) of the system, for example, according to a fixed or dynamic data communication schedule executed by the system. In other embodiments, the results of the in vivo monitored analyte level are not automatically communicated, transferred or output to one or more device or component of the system. In such embodiments, the results are provided only in response to a query to the system. That is, the results are communicated to a component or a device of the system only in response to the query or request for such results. In certain embodiments, the results of the in vivo monitoring may be logged or stored in a memory of the system and only communicated or transferred to another device or component of the system after the one or more predetermined monitoring time periods.

Embodiments include software and/or hardware to transform any one of the devices, components or systems into any one of the other devices, components or systems, where such transformation may be user-configurable after manufacture. Transformation modules that include hardware and/or software to accomplish such transformation may be mateable to a given system to transform it.

Embodiments include electronics coupled to analyte sensors that provide functionalities to operate the analyte sensors for monitoring analyte levels over a predetermined monitoring time period such as for example, about 30 days (or more in certain embodiments), about 14 days, about 10 days, about 5 days, about 1 day, less than about 1 day. In certain embodiments, the usage life of each analyte sensor may be the same as or different from the predetermined monitoring time periods. Components of the electronics to provide the functionalities to operate the analyte sensors in certain embodiments include control logic or microprocessors coupled to a power supply such as a battery to drive the in vivo analyte sensors to perform electrochemical reactions to generate resulting signals that correspond to the monitored analyte levels.

Electronics may also include other components such as one or more data storage units or memory (volatile and/or non volatile), communication component(s) to communicate information corresponding to the in vivo monitored analyte level to a display device automatically when the information is available, or selectively in response to a request for the monitored analyte level information. Data communication between display devices and the electronics units coupled to the sensor may be implemented serially (e.g., data transfer between them are not performed at the same time), or in parallel. For example, the display device may be configured to transmit a signal or data packet to the electronics coupled to the sensor, and upon receipt of the transmitted signal or data packet, the electronics coupled to the sensor communicates back to the display device. In certain embodiments, a display device may be configured to provide Radio Frequency (RF) power and data/signals continually, and detecting or receiving one or more return data packet or signal from electronics coupled to the sensor when it is within a predetermined RF power range from the display device. In certain embodiments, the display device and the electronics coupled to the sensor may be configured to transmit one or more data packets at the same time.

In certain embodiments, the one or more data storage units or memory stores data under the control of the electronics. In certain embodiments, the one or more data storage units or memory stores data according to a rolling data storage protocol executed by the control logic or microprocessors of the electronics. The data may be rolled according to time and/or prioritization, or otherwise. For example, a rolling data storage protocol may include a First-In/First-Out (FIFO) algorithm, First-In/Last-Out (FILO) algorithm, Last-In/First-Out (LIFO) algorithm, Last-In/Last-Out (LILO) algorithm. For example, embodiments include displacing the oldest stored data with most recent data in an iterative manner, or other rolling data protocol variations thereof.

Embodiments also include electronics programmed to store or log in the one or more data storage units or a memory data associated with the monitored analyte level over the sensor usage life or during a monitoring time period. During the monitoring time period, information corresponding to the monitored analyte level may be stored but not displayed or output during the sensor usage life, and the stored data may be later retrieved from memory at the end of the sensor usage life or after the expiration of the predetermined monitoring time period, e.g., for clinical analysis or therapy management.

In certain embodiments, the predetermined monitoring time period may be the same as the sensor usage life time period such that when an analyte sensor usage life expires (thus no longer used for in vivo analyte level monitoring), the predetermined monitoring time period ends. In certain other embodiments, the predetermined monitoring time period may include multiple sensor usage life time periods such that when an analyte sensor usage life expires, the predetermined monitoring time period has not ended, and the expired analyte sensor is replaced with another analyte sensor during the same predetermined monitoring time period. The predetermined monitoring time period may include the replacement of multiple analyte sensors for use.

In certain embodiments, in addition to the monitored analyte level information, other information may be communicated to a device, system or a component thereof, such as, but not limited to, monitored temperature information, heart rate, one or more biomarkers such as HbAlC or the like, stored analyte level information spanning a time period, e.g., the past 1 second to about 48 hours, e.g., the past 1 minute to about 24 hours, e.g., the past about 1 minute to about 10 hours, e.g., the past about 8 hours, or the past about 2 hours, or the past about 1 hour, or the past about 30 minutes, or the past about 15 minutes.

In certain embodiments, temperature (in vivo and/or skin and/or ambient) information may be obtained and stored in memory, e.g., to be used in an algorithm to compensate for temperature dependent inaccuracies in monitored analyte levels.

Embodiments include wirelessly communicating analyte level information from an on body electronics device to a second device such as a display device. Examples of communication protocols between on body electronics and the display device may include radio frequency identification (RFID) protocols or RF communication protocols. Exemplary RFID protocols include but are not limited to near field communication protocols that include short communication ranges (e.g., about 12 inches or less, or about 6 inches or less, or about 3 inches or less, or about 2 inches or less), high frequency wireless communication protocols, far field communication protocols (e.g., using ultra high frequency (UHF) communication systems) for providing signals or data from on body electronics to display devices.

Communication protocols may use 433 MHz frequency, 13.56 MHz frequency, 2.45 GHz frequency, or other suitable frequencies for wireless communication between the on body electronics that includes electronics coupled to an analyte sensor, and display devices and/or other devices such as a personal computer. While certain data transmission frequencies and/or data communication ranges are described above, within the scope of the present disclosure, other data suitable data transmission frequencies and/or data communication ranges may be used between the various devices in the analyte monitoring system.

Embodiments include data management systems including, for example, a data network and/or personal computer and/or a server terminal and/or one or more remote computers that are configured to receive collected or stored data from the display device for presenting analyte information and/or further processing in conjunction with the physiological monitoring for health management. For example, a display device may include one or more communication ports (hard wired or wireless) for connection to a data network or a computer terminal to transfer collected or stored analyte related data to another device and/or location. Analyte related data, in certain embodiments, are directly communicated from the electronics coupled to the analyte sensor to a personal computer, server terminal, and/or remote computers over the data network.

In certain embodiments, calibration "invisible" systems and methods are provided that determine clinically accurate analyte concentrations at least over the predetermined sensing period of analyte sensor systems without obtaining one or more independent analyte measurements (e.g., without using an in vitro test strip or other reference device) for calibration of generated analyte related signal from the analyte sensor during the usage life of the sensor, i.e., post-manufacture. In other words, once the analyte sensors are positioned in the body of the user, control logic or microprocessors in the electronics, or the microprocessors in the display device include one or more algorithms or programming to accurately convert or correlate signals related to the sensed analyte (e.g., in nA, counts, or other appropriate units) to a corresponding analyte level (e.g., converted to an analyte level in mg/dL or other appropriate units) without a reference value provided to the system, rendering sensor calibration "invisible" to the user such that the system does not require any human intervention for analyte sensor calibration.

These and other features, objects and advantages of the present disclosure will become apparent to those persons skilled in the art upon reading the details of the present disclosure as more fully described below.

### BRIEF DESCRIPTION OF THE DRAWINGS

FIG. 1 illustrates analyte monitoring system for real time analyte (e.g., glucose) measurement, data acquisition and/or processing in certain embodiments;
FIGS. 2A-2B are perspective and cross-sectional perspective views, respectively, of the housing including analyte sensor and on body electronics of system in FIG. 1 in certain embodiments;
FIGS. 3A-3C illustrate cross sectional perspective views of the thermistor assembly for on body electronics of FIG. 1 in certain embodiments;
FIGS. 4A and 4B are side cross sectional view and bottom planar views respectively, of the on body electronics with thermistor mounted in certain embodiments; and
FIG. 5 is a schematic of the on body electronics with guard traces surrounding the analyte sensor electrodes in certain embodiments.

### DETAILED DESCRIPTION

Before the present disclosure is described in detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges as also encompassed within the disclosure, subject to any specifically excluded limit in the state range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described. All publications mentioned herein to disclose and describe the methods and/or materials in connection with which the publications are cited.

It must be noted that as used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope of the present disclosure.

The figures shown herein are not necessarily drawn to scale, with some components and features being exaggerated for clarity.

Generally, embodiments of the present disclosure relate to in vivo methods and devices for detecting at least one analyte such as glucose in body fluid. Accordingly, embodiments include in vivo analyte sensors configured so that at least a portion of the sensor is positioned in the body of a user (e.g., within the ISF), to obtain information about at least one analyte of the body, e.g., transcutaneously positioned in user's body. In certain embodiments, an in vivo analyte sensor is coupled to an electronics unit that is maintained on the body of the user such as on a skin surface, where such coupling provides on body, in vivo analyte sensor electronics assemblies.

In certain embodiments, analyte information is communicated from a first device such as an on body electronics unit to a second device which may include user interface features, including a display, and/or the like. Information may be communicated from the first device to the second device automatically and/or continuously when the analyte information is available, or may not be communicated automatically and/or continuously, but rather stored or logged in a memory of the first device. Accordingly, in many embodiments of the system, analyte information derived by the sensor/on body electronics (for example, on body electronics assembly) is made available in a user-usable or viewable form only when queried by the user such that the timing of data communication is selected by the user.

In this manner, analyte information is only provided or evident to a user (provided at a user interface device) when desired by the user even though an in vivo analyte sensor automatically and/or continuously monitors the analyte level in vivo, i.e., the sensor automatically monitors analyte such as glucose on a pre-defined time interval over its usage life. For example, an analyte sensor may be positioned in vivo and coupled to on body electronics for a given sensing period, e.g., about 14 days. In certain embodiments, the sensor-derived analyte information is automatically communicated from the sensor electronics assembly to a remote monitor device or display device for output to a user throughout the 14 day period according to a schedule programmed at the on body electronics (e.g., about every 1 minute or about every 5 minutes or about every 10 minutes, or the like). In certain embodiments, sensor-derived analyte information is only communicated from the sensor electronics assembly to a remote monitor device or display device at user-determined times, e.g., whenever a user decides to check analyte information. At such times, a communications system is activated and sensor-derived information is then sent from the on body electronics to the remote device or display device.

In still other embodiments, the information may be communicated from the first device to the second device automatically and/or continuously when the analyte information is available, and the second device stores or logs the received information without presenting or outputting the information to the user. In such embodiments, the information is received by the second device from the first device when the information becomes available (e.g., when the sensor detects the analyte level according to a time schedule). However, the received information is initially stored in the second device and only output to a user interface or an output component of the second device (e.g., display) upon detection of a request for the information on the second device.

Accordingly, in certain embodiments once a sensor electronics assembly is placed on the body so that at least a portion of the in vivo sensor is in contact with bodily fluid such as ISF and the sensor is electrically coupled to the electronics unit, sensor derived analyte information may be communicated from the on body electronics to a display device on-demand by powering on the display device (or it may be continually powered), and executing a software algorithm stored in and accessed from a memory of the display device, to generate one or more request commands, control signal or data packet to send to the on body electronics. The software algorithm executed under, for example, the control of the microprocessor or application specific integrated circuit (ASIC) of the display device may include routines to detect the position of the on body electronics relative to the display device to initiate the transmission of the generated request command, control signal and/or data packet.

Display devices may also include programming stored in memory for execution by one or more microprocessors and/or ASICs to generate and transmit the one or more request command, control signal or data packet to send to the on body electronics in response to a user activation of an input mechanism on the display device such as depressing a button on the display device, triggering a soft button associated with the data communication function, and so on. The input mechanism may be alternatively or additionally provided on or in the on body electronics which may be configured for user activation. In certain embodiments, voice commands or audible signals may be used to prompt or instruct the microprocessor or ASIC to execute the software routine(s) stored in the memory to generate and transmit the one or more request command, control signal or data packet to the on body device. In the embodiments that are voice activated or responsive to voice commands or audible signals, on body electronics and/or display device includes a microphone, a speaker, and processing routines stored in the respective memories of the on body electronics and/or the display device to process the voice commands and/or audible signals. In certain embodiments, positioning the on body device and the display device within a predetermined distance (e.g., close proximity) relative to each other initiates one or more software routines stored in the memory of the display device to generate and transmit a request command, control signal or data packet.

As described, embodiments include in vivo analyte sensors and on body electronics that together provide body wearable sensor electronics assemblies. In certain embodiments, in vivo analyte sensors are fully integrated with on body electronics (fixedly connected during manufacture), while in other embodiments they are separate but connectable post manufacture (e.g., before, during or after sensor insertion into a body). On body electronics may include an in vivo glucose sensor, electronics, battery, and antenna encased (except for the sensor portion that is for in vivo positioning) in a waterproof housing that includes or is attachable to an adhesive pad. In certain embodiments, the housing withstands immersion in about one meter of water for up to at least 30 minutes. In certain embodiments, the housing withstands continuous underwater contact, e.g., for longer than about 30 minutes, and continues to function properly according to its intended use, e.g., without water damage to the housing electronics where the housing is suitable for water submersion.

Embodiments include portable handheld display devices, as separate devices and spaced apart from an on body electronics assembly, that collect information from the assemblies and provide sensor derived analyte readings to users. Such devices may also be referred to as meters, readers, monitors, receivers, human interface devices, companions, or the like. Certain embodiments may include an integrated in vitro analyte meter. In certain embodiments, display devices include one or more wired or wireless communications ports such as USB, serial, parallel, or the like, configured to establish communication between a display device and another unit (e.g., on body electronics, power unit to recharge a battery, a PC, etc). For example, a display device communication port may enable charging a display device battery with a respective charging cable and/or data exchange between a display device and its compatible informatics software.

Compatible informatics software in certain embodiments include, for example, but not limited to stand alone or network connection enabled data management software program, resident or running on a display device, personal computer, a server terminal, for example, to perform data analysis, charting, data storage, data archiving and data communication as well as data synchronization. Informatics software, in certain embodiments, may also include software for executing field upgradable functions to upgrade firmware of a display device and/or on body electronics unit to upgrade the resident software on the display device and/or the on body electronics unit, e.g., with versions of firmware that include additional features and/or include software bugs or errors fixed, etc.

Embodiments of the subject disclosure are described primarily with respect to glucose monitoring devices and systems, and methods of glucose monitoring, for convenience only and such description is in no way intended to limit the scope of the disclosure. It is to be understood that the analyte monitoring system may be configured to monitor a variety of analytes at the same time or at different times.

For example, analytes that may be monitored include, but are not limited to, acetyl choline, amylase, bilirubin, cholesterol, chorionic gonadotropin, creatine kinase (e.g. , CK- MB), creatine, DNA, fructosamine, glucose, glutamine, growth hormones, hormones, ketones, lactate, oxygen, peroxide, prostate-specific antigen, prothrombin, R A, thyroid stimulating hormone, and troponin. The concentration of drugs, such as, for example, antibiotics (e.g. , gentamicin, vancomycin, and the like), digitoxin, digoxin, drugs of abuse, theophylline, and warfarin, may also be monitored. In those embodiments that monitor more than one analyte, the analytes may be monitored at the same or different times, with a single sensor or with a plurality of sensors which may use the same on body electronics (e.g., simultaneously) or with different on body electronics.

For example, a predetermined monitoring time period may begin with positioning the sensor in vivo and in contact with a body fluid such as ISF, and/or with the initiation (or powering on to full operational mode) of the on body electronics. Initialization of on body electronics may be implemented with a command generated and transmitted by a display device in response to the activation of a switch and/or by placing the display device within a predetermined distance (e.g., close proximity) to the on body electronics, or by user manual activation of a switch on the on body electronics unit, e.g., depressing a button, or such activation may be caused by the insertion device, e.g., as described in U.S. Patent Application No. 12/698,129 filed on February 1 , 2010 and published as US-A-2010/0324392..

When initialized in response to a received command from a display device, the on body electronics retrieves and executes from its memory software routine to fully power on the components of the on body electronics, effectively placing the on body electronics in full operational mode in response to receiving the activation command from the display device. For example, prior to the receipt of the command from the display device, a portion of the components in the on body electronics may be powered by its internal power supply such as a battery while another portion of the components in the on body electronics may be in powered down or low power including no power, inactive mode, or all components may be in an inactive mode, powered down mode. Upon receipt of the command, the remaining portion (or all) of the components of the on body electronics is switched to active, fully operational mode.

Embodiments of on body electronics may include one or more printed circuit boards with electronics including control logic implemented in ASIC, microprocessors, memory, and the like, and transcutaneously positionable analyte sensors forming a single assembly. On body electronics may be configured to provide one or more signals or data packet associated with a monitored analyte level upon detection of a display device of the analyte monitoring system within a predetermined proximity for a period of time (for example, about 2 minutes or less, e.g., about 1 minute or less, e.g., about 30 seconds or less, e.g., about 10 seconds or less, e.g., about 5 seconds or less, e.g., about 2 seconds or less) and/or until a confirmation, such as an audible and/or visual and/or tactile (e.g., vibratory) notification, is output on the display device indicating successful acquisition of the analyte related signal from the on body electronics. A distinguishing notification may also be output for unsuccessful acquisition in certain embodiments.

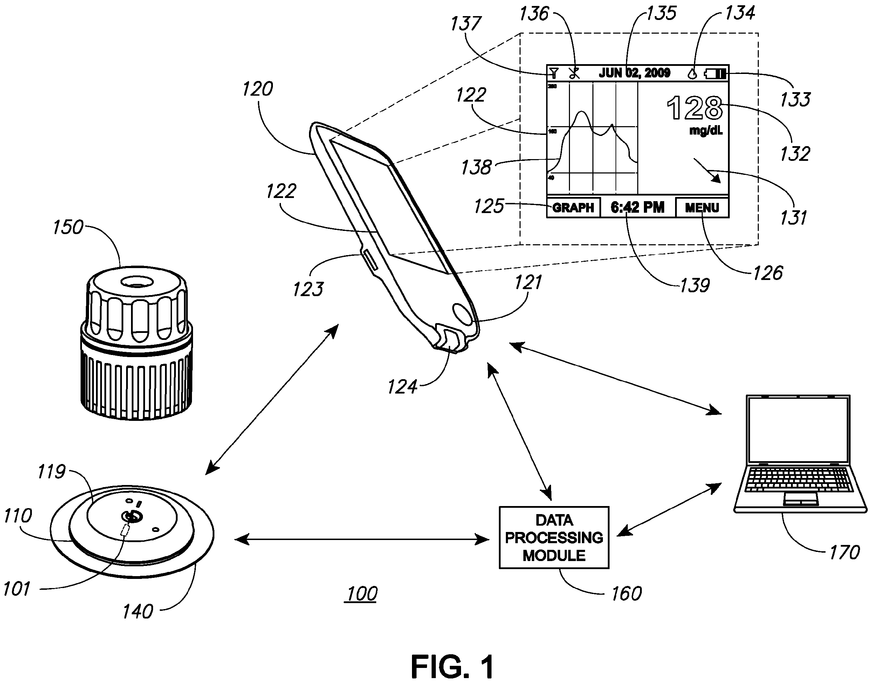

FIG. 1 shows an exemplary in vivo-based analyte monitoring system 100 in accordance with embodiments of the present disclosure. As shown, in certain embodiments, analyte monitoring system 100 includes on body electronics 110 electrically coupled to in vivo analyte sensor 101 (a proximal portion of which is shown in FIG. 1) and attached to adhesive layer 140 for attachment on a skin surface on the body of a user. On body electronics 110 includes on body housing 119, that defines an interior compartment. Also shown in FIG. 1 is insertion device 150 that, when operated, transcutaneously positions a portion of analyte sensor 101 through a skin surface and in fluid contact with ISF , and positions on body electronics 110 and adhesive layer 140 on a skin surface. In certain embodiments, on body electronics 110, analyte sensor 101 and adhesive layer 140 are sealed within the housing of insertion device 150 before use, and in certain embodiments, adhesive layer 140 is also sealed within the housing or itself provides a terminal seal of the insertion device 150.

Referring back to the FIG. 1, analyte monitoring system 100 includes display device 120 which includes a display 122 to output information to the user, an input component 121 such as a button, actuator, a touch sensitive switch, a capacitive switch, pressure sensitive switch, jog wheel or the like, to input data or command to display device 120 or otherwise control the operation of display device 120. It is noted that some embodiments may include display-less devices or devices without any user interface components. These devices may be functionalized to store data as a data logger and/or provide a conduit to transfer data from on body electronics and/or a display-less device to another device and/or location. Embodiments will be described herein as display devices for exemplary purposes which are in no way intended to limit the embodiments of the present disclosure. It will be apparent that display-less devices may also be used in certain embodiments.

In certain embodiments, on body electronics 110 may be configured to store some or all of the monitored analyte related data received from analyte sensor 101 in a memory during the monitoring time period, and maintain it in memory until the usage period ends. In such embodiments, stored data is retrieved from on body electronics 110 at the conclusion of the monitoring time period, for example, after removing analyte sensor 101 from the user by detaching on body electronics 110 from the skin surface where it was positioned during the monitoring time period. In such data logging configurations, real time monitored analyte level is not communicated to display device 120 during the monitoring period or otherwise transmitted from on body electronics 110, but rather, retrieved from on body electronics 110 after the monitoring time period.

In certain embodiments, input component 121 of display device 120 may include a microphone and display device 120 may include software configured to analyze audio input received from the microphone, such that functions and operation of the display device 120 may be controlled by voice commands. In certain embodiments, an output component of display device 120 includes a speaker for outputting information as audible signals. Similar voice responsive components such as a speaker, microphone and software routines to generate, process and store voice driven signals may be provided to on body electronics 110.

In certain embodiments, display 122 and input component 121 may be integrated into a single component, for example a display that can detect the presence and location of a physical contact touch upon the display such as a touch screen user interface. In such embodiments, the user may control the operation of display device 120 by utilizing a set of pre-programmed motion commands, including, but not limited to, single or double tapping the display, dragging a finger or instrument across the display, motioning multiple fingers or instruments toward one another, motioning multiple fingers or instruments away from one another, etc. In certain embodiments, a display includes a touch screen having areas of pixels with single or dual function capacitive elements that serve as LCD elements and touch sensors.

Display device 120 also includes data communication port 123 for wired data communication with external devices such as remote terminal (personal computer) 170, for example. Example embodiments of the data communication port 123 include USB port, mini USB port, RS-232 port, Ethernet port, Firewire port, or other similar data communication ports configured to connect to the compatible data cables. Display device 120 may also include an integrated in vitro glucose meter, including in vitro test strip port 124 to receive an in vitro glucose test strip for performing in vitro blood glucose measurements.

Referring still to FIG. 1, display 122, in certain embodiments, is configured to display a variety of information - some or all of which may be displayed at the same or different time on display 122. In certain embodiments the displayed information is user-selectable so that a user can customize the information shown on a given display screen. Display 122 may include, but is not limited to, graphical display 138, for example, providing a graphical output of glucose values over a monitored time period (which may show important markers such as meals, exercise, sleep, heart rate, blood pressure, etc), numerical display 132, for example, providing monitored glucose values (acquired or received in response to the request for the information), and trend or directional arrow display 131 that indicates a rate of analyte change and/or a rate of the rate of analyte change, e.g., by moving locations on display 122.

As further shown in FIG. 1, display 122 may also include date display 135 providing for example, date information for the user, time of day information display 139 providing time of day information to the user, battery level indicator display 133 which graphically shows the condition of the battery (rechargeable or disposable) of the display device 120, sensor calibration status icon display 134 for example, in monitoring systems that require periodic, routine or a predetermined number of user calibration events, notifying the user that the analyte sensor calibration is necessary, audio/vibratory settings icon display 136 for displaying the status of the audio/vibratory output or alarm state, and wireless connectivity status icon display 13 7 that provides indication of wireless communication connection with other devices such as on body electronics, data processing module 160, and/or remote terminal 170. As additionally shown in FIG. 1, display 122 may further include simulated touch screen button 125, 126 for accessing menus, changing display graph output configurations or otherwise for controlling the operation of display device 120.

Referring back to FIG. 1, in certain embodiments, display 122 of display device 120 may be additionally, or instead of visual display, configured to output alarms notifications such as alarm and/or alert notifications, glucose values etc, which may be audible, tactile, or any combination thereof. In one aspect, the display device 120 may include other output components such as a speaker, vibratory output component and the like to provide audible and/or vibratory output indication to the user in addition to the visual output indication provided on display 122.

After the positioning of on body electronics 110 on the skin surface and analyte sensor 101 in vivo to establish fluid contact with ISF (or other appropriate body fluid), on body electronics 110 in certain embodiments is configured to wirelessly communicate analyte related data (such as, for example, data corresponding to monitored analyte level and/or monitored temperature data, and/or stored historical analyte related data) when on body electronics 110 receives a command or request signal from display device 120. In certain embodiments, on body electronics 110 may be configured to at least periodically broadcast real time data associated with monitored analyte level which is received by display device 120 when display device 120 is within communication range of the data broadcast from on body electronics 110, i.e., it does not need a command or request from a display device to send information.

For example, display device 120 may be configured to transmit one or more commands to on body electronics 110 to initiate data transfer, and in response, on body electronics 110 may be configured to wirelessly transmit stored analyte related data collected during the monitoring time period to display device 120. Display device 120 may in turn be connected to a remote terminal 170 such as a personal computer and functions as a data conduit to transfer the stored analyte level information from the on body electronics 110 to remote terminal 170. In certain embodiments, the received data from the on body electronics 110 may be stored (permanently or temporarily) in one or more memory of the display device 120. In certain other embodiments, display device 120 is configured as a data conduit to pass the data received from on body electronics 110 to remote terminal 170 that is connected to display device 120.

Referring still to FIG. 1, also shown in analyte monitoring system 100 are data processing module 160 and remote terminal 170. Remote terminal 170 may include a personal computer, a server terminal a laptop computer or other suitable data processing devices including software for data management and analysis and communication with the components in the analyte monitoring system 100. For example, remote terminal 170 may be connected to a local area network (LAN), a wide area network (WAN), or other data network for uni-directional or bi-directional data communication between remote terminal 170 and display device 120 and/or data processing module 160.

Remote terminal 170, in certain embodiments, may include one or more computer terminals located at a physician's office or a hospital. For example, remote terminal 170 may be located at a location other than the location of display device 120. Remote terminal 170 and display device 120 could be in different rooms or different buildings. Remote terminal 170 and display device 120 could be at least about one mile apart, e.g., at least about 10 miles apart, e.g., at least about 100 miles apart. For example, remote terminal 170 could be in the same city as display device 120, remote terminal 170 could be in a different city than display device 120, remote terminal 170 could be in the same state as display device 120, remote terminal 170 could be in a different state than display device 120, remote terminal 170 could be in the same country as display device 120, or remote terminal 170 could be in a different country than display device 120.

In certain embodiments, a separate, optional data communication/processing device such as data processing module 160 may be provided in analyte monitoring system 100. Data processing module 160 may include components to communicate using one or more wireless communication protocols such as, for example, but not limited to, infrared (IR) protocol, Bluetooth® protocol, Zigbee® protocol, and 802.11 wireless LAN protocol. Additional description of communication protocols including those based on Bluetooth® protocol and/or Zigbee® protocol can be found in U.S. Patent Publication No. 2006/0193375 . Data processing module
160 may further include communication ports, drivers or connectors to establish wired communication with one or more of display device 120, on body electronics 110, or remote terminal 170 including, for example, but not limited to USB connector and/or USB port, Ethernet connector and/or port, Fire Wire connector and/or port, or RS-232 port and/or connector.

In certain embodiments, and discussed above, data processing module 160 includes a Bluetooth® communication module that can be paired with one or more devices in the analyte monitoring system described in FIG. 1 that also has a Bluetooth® communication module. Upon pairing, data can be exchanged between paired devices. For example, in certain embodiments on body electronics 110 is provided with a Bluetooth® communication module that can be paired with the data processing module 160. In this case, the data processing module 160 may be configured to receive or otherwise programmed to request or prompt for monitored analyte data from the on body electronics 110 based either on a programmed data transmission or request schedule, periodically, or when desired (for example, pairing the devices so that the data processing module 160 is capable of requesting the glucose data from the on body electronics 110 either upon pairing, or upon sending a data request). In certain embodiments, the display device 120 includes a Bluetooth® communication module that is paired with the on body electronics 110 to receive or request for information related to the monitored glucose data from the on body electronics 110 either based on a programmed data transfer schedule, or upon activation of a command or request from the display device 120 communicated to the on body electronics 110.

In certain embodiments, data processing module 160 is programmed to transmit a polling or query signal to on body electronics 110 at a predetermined time interval (e.g., once every minute, once every five minutes, or the like), and in response, receive the monitored analyte level information from on body electronics 110. Data processing module 160 stores in its memory the received analyte level information, and/or relays or retransmits the received information to another device such as display device 120. More specifically in certain embodiments, data processing module 160 may be configured as a data relay device to retransmit or pass through the received analyte level data from on body electronics 110 to display device 120 or a remote terminal (for example, over a data network such as a cellular or WiFi data network) or both.

In certain embodiments, on body electronics 110 and data processing module 160 may be positioned on the skin surface of the user within a predetermined distance of each other (for example, about 1-12 inches, or about 1-10 inches, or about 1-7 inches, or about 1-5 inches) such that periodic communication between on body electronics 110 and data processing module 160 is maintained. Alternatively, data processing module 160 may be worn on a belt or clothing item of the user, such that the desired distance for communication between the on body electronics 110 and data processing module 160 for data communication is maintained. In a further aspect, the housing of data processing module 160 may be configured to couple to or engage with on body electronics 110 such that the two devices are combined or integrated as a single assembly and positioned on the skin surface. In further embodiments, data processing module 160 is detachably engaged or connected to on body electronics 110 providing additional modularity such that data processing module 160 may be optionally removed or reattached as desired.

Referring again to FIG. 1, in certain embodiments, data processing module 160 is programmed to transmit a command or signal to on body electronics 110 at a predetermined time interval such as once every minute, or once every 5 minutes or once every 30 minutes or any other suitable or desired programmable time interval to request analyte related data from on body electronics 110. When data processing module 160 receives the requested analyte related data, it stores the received data. In this manner, analyte monitoring system 100 may be configured to receive the continuously monitored analyte related information at the programmed or programmable time interval, which is stored and/or displayed to the user. The stored data in data processing module 160 may be subsequently provided or transmitted to display device 120, remote terminal 170 or the like for subsequent data analysis such as identifying frequency of periods of glycemic level excursions over the monitored time period, or the frequency of the alarm event occurrence during the monitored time period, for example, to improve therapy related decisions. Using this information, the doctor, healthcare provider or the user may adjust or recommend modification to the diet, daily habits and routines such as exercise, and the like.

In another embodiment, data processing module 160 transmits a command or signal to on body electronics 110 to receive the analyte related data in response to a user activation of a switch provided on data processing module 160 or a user initiated command received from display device 120. In further embodiments, data processing module 160 is configured to transmit a command or signal to on body electronics 110 in response to receiving a user initiated command only after a predetermined time interval has elapsed. For example, in certain embodiments, if the user does not initiate communication within a programmed time period, such as, for example, about 5 hours from last communication (or 10 hours from the last communication, or 24 hours from the last communication), the data processing module 160 may be programmed to automatically transmit a request command or signal to on body electronics 110. Alternatively, data processing module 160 may be programmed to activate an alarm to notify the user that a predetermined period of time has elapsed since the last communication between the data processing module 160 and on body electronics 110. In this manner, users or healthcare providers may program or configure data processing module 160 to provide certain compliance with analyte monitoring regimen, so that frequent determination of analyte levels is maintained or performed by the user.

In certain embodiments, when a programmed or programmable alarm condition is detected (for example, a detected glucose level monitored by analyte sensor 101 that is outside a predetermined acceptable range indicating a physiological condition which requires attention or intervention for medical treatment or analysis (for example, a hypoglycemic condition, a hyperglycemic condition, an impending hyperglycemic condition or an impending hypoglycemic condition), the one or more output indications may be generated by the control logic or processor of the on body electronics 110 and output to the user on a user interface of on body electronics 110 so that corrective action may be timely taken. In addition to or alternatively, if display device 120 is within communication range, the output indications or alarm data may be communicated to display device 120 whose processor, upon detection of the alarm data reception, controls the display 122 to output one or more notification.

In certain embodiments, the data processing module 160 may include a user interface to provide the same or similar user interaction and data input/output options as provided in the display device 120, obviating the need for the display device 120. In other words, the functionality of the display device 120 may be incorporated into the data processing module 160 such that the user interacts with the data processing module 160 to monitor the analyte levels and receive notifications or alerts or alarms associated with the monitored analyte levels.

In certain embodiments, control logic or microprocessors of on body electronics 110 include software programs to determine future or anticipated analyte levels based on information obtained from analyte sensor 101, e.g., the current analyte level, the rate of change of the analyte level, the acceleration of the analyte level change, and/or analyte trend information determined based on stored monitored analyte data providing a historical trend or direction of analyte level fluctuation as function time during monitored time period. Predictive alarm parameters may be programmed or programmable in display device 120, or the on body electronics 110, or both, and output to the user in advance of anticipating the user's analyte level reaching the future level. This provides the user an opportunity to take timely corrective action.

Information, such as variation or fluctuation of the monitored analyte level as a function of time over the monitored time period providing analyte trend information, for example, may be determined by one or more control logic or microprocessors of display device 120, data processing module 160, and/or remote terminal 170, and/or on body electronics 110. Such information may be displayed as, for example, a graph (such as a line graph) to indicate to the user the current and/or historical and/or predicted future analyte levels as measured and predicted by the analyte monitoring system 100. Such information may also be displayed as directional arrows (for example, see trend or directional arrow display 131) or other icon(s), e.g., the position of which on the screen relative to a reference point indicated whether the analyte level is increasing or decreasing as well as the acceleration or deceleration of the increase or decrease in analyte level. This information may be utilized by the user to determine any necessary corrective actions to ensure the analyte level remains within an acceptable and/or clinically safe range. Other visual indicators, including colors, flashing, fading, etc., as well as audio indicators including a change in pitch, volume, or tone of an audio output and/or vibratory or other tactile indicators may also be incorporated into the display of trend data as means of notifying the user of the current level and/or direction and/or rate of change of the monitored analyte level. For example, based on a determined rate of glucose change, programmed clinically significant glucose threshold levels (e.g., hyperglycemic and/or hypoglycemic levels), and current analyte level derived by an in vivo analyte sensor, the system 100 may include an algorithm stored on computer readable medium to determine the time it will take to reach a clinically significant level and will output notification in advance ofreaching the clinically significant level, e.g., 30 minutes before a clinically significant level is anticipated, and/or 20 minutes, and/or 10 minutes, and/or 5 minutes, and/or 3 minutes, and/or 1 minute, and so on, with outputs increasing in intensity or the like.

In certain embodiments, data processing module 160 may include a data storage unit or a memory device such as an electrically erasable programmable read only memory (EEPROM) that includes pre-programmed parameters associated with the operation of the on body electronics 110. For example, the memory device of the data processing module 160 may be programmed to include or store a security key at a specific memory address that is retrieved or read by the processing unit of the data processing module 160, while other memory locations may store or point to address locations that include on body electronics data processing parameters, sensor sensitivity information, sensor expiration data, alarm or alert threshold levels, parameters for determining rate of change or trend information associated with the monitored analyte level and the like. Other parameters stored in the memory device to be read and one or more routines called and executed by the processing unit of the data processing module 160 includes supported data communication such as Bluetooth® data communication, wired or cabled communication with other devices such as the remote terminal 170, the display device 120, mobile smart phones and the like.

Referring again back to FIG. 1, in certain embodiments, software algorithm(s) for execution by data processing module 160 may be stored in an external memory device such as an SD card, microSD card, compact flash card, XD card, Memory Stick card, Memory Stick Duo card, or USB memory stick/device including executable programs stored in such devices for execution upon connection to the respective one or more of the on body electronics 110, remote terminal 170 or display device 120. In a further aspect, software algorithms for execution by data processing module 160 may be provided to a communication device such as a mobile telephone including, for example, WiFi or Internet enabled smart phones or personal digital assistants (PDAs) as a downloadable application for execution by the downloading communication device.

Examples of smart phones include Windows®, Android™, iPhone® operating system, Palm® WebOS™, Blackberry® operating system, or Symbian® operating system based mobile telephones with data network connectivity functionality for data communication over an internet connection and/or a local area network (LAN). PDAs as described above include, for example, portable electronic devices including one or more microprocessors and data communication capability with a user interface (e.g., display/output unit and/or input unit, and configured for performing data processing, data upload/download over the internet, for example. In such embodiments, remote terminal 170 may be configured to provide the executable application software to the one or more of the communication devices described above when communication between the remote terminal 170 and the devices are established.

In still further embodiments, executable software applications may be provided over-the-air (OTA) as an OTA download such that wired connection to remote terminal 170 is not necessary. For example, executable applications may be automatically downloaded as software download to the communication device, and depending upon the configuration of the communication device, installed on the device for use automatically, or based on user confirmation or acknowledgement on the communication device to execute the installation of the application. The OTA download and installation of software may include software applications and/or routines that are updates or upgrades to the existing functions or features of data processing module 160 and/or display device 120.

Referring back to remote terminal 170 of FIG. 1, in certain embodiments, new software and/or software updates such as software patches or fixes, firmware updates or software driver upgrades, among others, for display device 120 and/or on body electronics 110 and/or data processing module 160 may be provided by remote terminal 170 when communication between the remote terminal 170 and display device 120 and/or data processing module 160 is established. For example, software upgrades, executable programming changes or modification for on body electronics 110 may be received from remote terminal 170 by one or more of display device 120 or data processing module 160, and thereafter, provided to on body electronics 110 to update its software or programmable functions. For example, in certain embodiments, software received and installed in on body electronics 110 may include software bug fixes, modification to the previously installed software parameters (modification to analyte related data storage time interval, resetting or adjusting time base or information of on body electronics 110, modification to the transmitted data type, data transmission sequence, or data storage time period, among others).

In certain embodiments, when the display device 120 is connected to the remote terminal 170, and the data management software executed on the remote terminal 170 determines that an upgrade to the software in the display device 120 and/or the on body electronics 110 is available, the remote terminal 170, based either upon user acceptance/initiation or automatically, downloads or retrieves the available software upgrade which in one embodiment, includes an upgrade tool that is downloaded and executed to initiate the software upgrade routine. In certain embodiments, the downloaded software upgrade tool is a separate executable program independent of the data management software executed on the remote terminal 170, and which, upon execution, performs the software upgrade independent of the resident data management software.

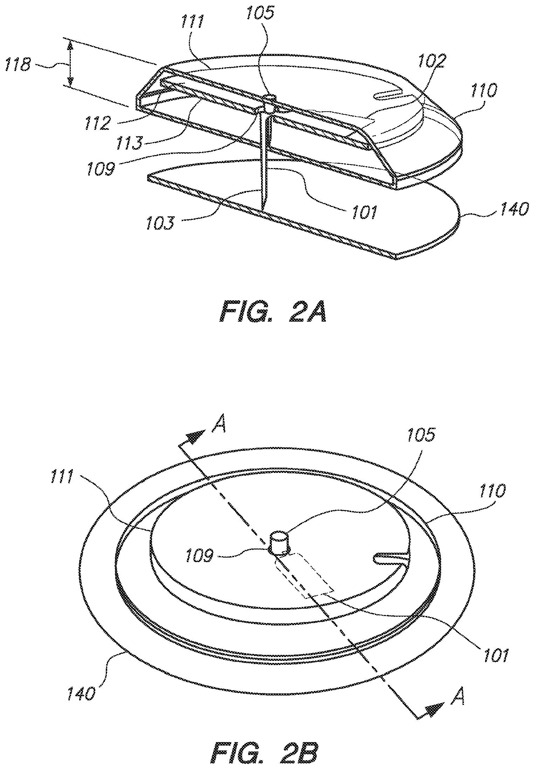

FIGS. 2A-2B are perspective and top cross sectional views, respectively, of on body electronics 110 of FIG. 1 in certain embodiments. In particular, FIG. 2A illustrates the cross-sectional view of on body electronics 110 along the dotted line A shown in FIG. 2B. Referring to FIGS. 2A-2B, on body electronics 110 in certain embodiments is sized and shaped such that the height or thickness profile is minimized (for example, to less than or equal to about 10 mm, e.g., or less than or equal to about 7 mm, e.g., or less than or equal to about 5 mm, e.g., or less than or equal to about 4.5 mm, e.g., or less than or equal to about 4 mm or less). For example, as shown in the figures, in certain embodiments, on body electronics 110 includes a dome-like or tapered shape with a height or thickness dimension of up to about 5 mm at its thickest point, and may taper (gradually or step wise) to a height or thickness dimension of less than about 4 mm, or about 3mm or less, or about 2 mm or less, or about 1 mm or less. In certain embodiments, on body electronics 110 has a compact z-height 118 (e.g., height or thickness of on body electronics 110) that is not more than about 4.5mm thick at its thickest area (if the thickness is not uniform or rather varies within a given unit), and no more than about 4.6 mm thick including an adhesive patch.

Referring to FIGS. 2A-2B, in certain embodiments, analyte sensor 101 is assembled during manufacturing with on body electronics 110, for example, and fixedly connected to PCB 111 of on body electronics 110. As shown in FIGS. 2A-2B, proximal portion 102 of sensor 101 is placed on upper surface 112 of PCB 111 and secured to PCB 111 for example, using rivets, fasteners, clamps or the like. The fixedly positioned proximal portion 102 of sensor 101 may be positioned such that proximal portion 102 is electrically coupled to the respective contact points on upper surface 112 of PCB 111. As can be further seen from FIGS. 2A-2B, in such embodiments, the distal portion 103 of sensor 101 is bent or angled such that approximately a 90 degree angle is defined between the proximal portion 102 and distal portion 103 of sensor 101. In certain embodiments, the angle between the proximal portion 102 and distal portion 103 of sensor 101 may be less than about 90 degrees, less than about 80 degrees, less than about 70 degrees, less than about 60 degrees, less than about 50 degrees, less than about 40 degrees, less than about 30 degrees, less than about 20 degrees, or less than about 10 degrees.

Referring still to FIG. 2A-2B, as shown, sensor 101 is positioned relative to PCB 111 such that sensor 101 is positioned through opening 109 defined between upper surface 112 and lower surface 113 of PCB 111. In certain embodiments, PCBs of on body electronics do not include an opening such as that shown in FIGS. 2A-2B.

Furthermore, adhesive layer 140 (single sided or two sided) may be provided to securely position on body electronics 110 on the skin surface during and after sensor deployment. Adhesive may be manufactured so to be attached to the on body unit, or to be attachable post manufacturing, e.g., by a user. In certain embodiments, a sensor insertion process causes the adhesive patch to be attached to the on body unit. In certain embodiments, on body electronics 110 with analyte sensor 101 may be contained or disposed (e.g., during manufacturing) within insertion device 150 (FIG. 1), avoiding the need for a user to align, position, or otherwise connect or couple analyte sensor 101 and on body electronics 110 to insertion device 150 (FIG. 1) prior to the insertion of analyte sensor 101 and initializing on body electronics 110. In certain embodiments, an optional sensor guide 105 is provided to further assist in alignment of the analyte sensor 101 with insertion device 150. Thus, potential misuse, user error, or misalignment of analyte sensor 101 relative to a needle or insertion mechanism of insertion device 150 by the user may be avoided.

Referring to FIGS. 2A-2B, embodiments of on body electronics 110 include dimensions and weight that are optimized for reduction and thus maximized for comfort in use and wear. In certain embodiments, on body electronics 110 has a small on-body footprint, e.g., less than about 50 mm in diameter excluding adhesive patch 140 e.g., less than about 45 mm in diameter excluding adhesive patch 140, e.g., less than about 40 mm in diameter excluding adhesive patch 140, e.g., less than about 35 mm in diameter excluding adhesive patch 140, e.g., less than about 30 mm in diameter excluding adhesive patch 140, where in certain embodiments the on-body footprint may be about 25 mm to about 28 mm excluding adhesive patch 140.

In certain embodiments, on body electronics 110, including adhesive patch 140, has an on-body footprint that is less than about 70 mm in diameter (at its widest if isn't uniform), e.g., less than about 65 mm in diameter, e.g., less than about 60 mm in diameter, e.g., less than about 55 mm in diameter, e.g., less than about 50 mm in diameter, e.g., less than about 45 mm in diameter, e.g., less than about 40 mm in diameter, where in certain embodiments the on-body footprint may be about 35 mm to about 37 mm including adhesive patch 140.

In certain embodiments, adhesive patch 140 has an on body footprint that is less than about 3.0 inches in diameter, e.g., less than about 2.0 inches in diameter, less than about 1.0 inch in diameter, where, in certain embodiments, an adhesive patch may have a diameter that is 1.0 inch to about 1.5 inches or less.

Embodiments include on body electronics 110 that has a small surface area, e.g., less than about 2 square inches excluding adhesive patch 140, e.g., less than about 1.5 square inches excluding adhesive patch 140, e.g., less than about 1 square inch excluding adhesive patch 140, e.g., less than about 0.9 square inches excluding adhesive patch 140, e.g., less than about 0.8 square inches excluding adhesive patch 140, e.g., less than about 0.75 square inches excluding adhesive patch 140, e.g., less than about .7 square inches excluding adhesive patch 140, where in certain embodiments the surface area of an on body electronics unit may be about 0.75 square inches to about 0.79 square inches excluding adhesive patch 140.

In certain embodiments, on body electronics 110, including adhesive patch 140, has a surface area that is about 3.0 square inches or less including an adhesive patch, e.g., about 2.0 square inches or less including an adhesive patch, e.g., about 1.9 square inches or less including an adhesive patch, e.g., about 1.8 square inches or less including an adhesive patch, e.g., about 1.75 square inches or less including an adhesive patch, e.g., about 1.6 square inches or less including an adhesive patch, where in certain embodiments the surface area of an on body electronics unit may be about 1.7 5 square inches to about 1.77 square inches or less.

In certain embodiments, on body electronics 110 may have a circular footprint and/or adhesive patch 140 may have a circular footprint. In certain embodiments, on body electronics 110 may be circular in shape. Other shapes for on body electronics and/or adhesive patches include, but are not limited to, oval, rectangle, square triangle, or polygon shapes, as well as irregular and complex shapes.

In certain embodiments, on body electronics 110 has low mass, e.g., less than about 10 grams including adhesive patch 140 e.g., less than about 5 grams including adhesive patch 140, less than about 3.5 grams including adhesive patch 140, where, in certain embodiments the mass is no more than 3 grams including adhesive patch 140.

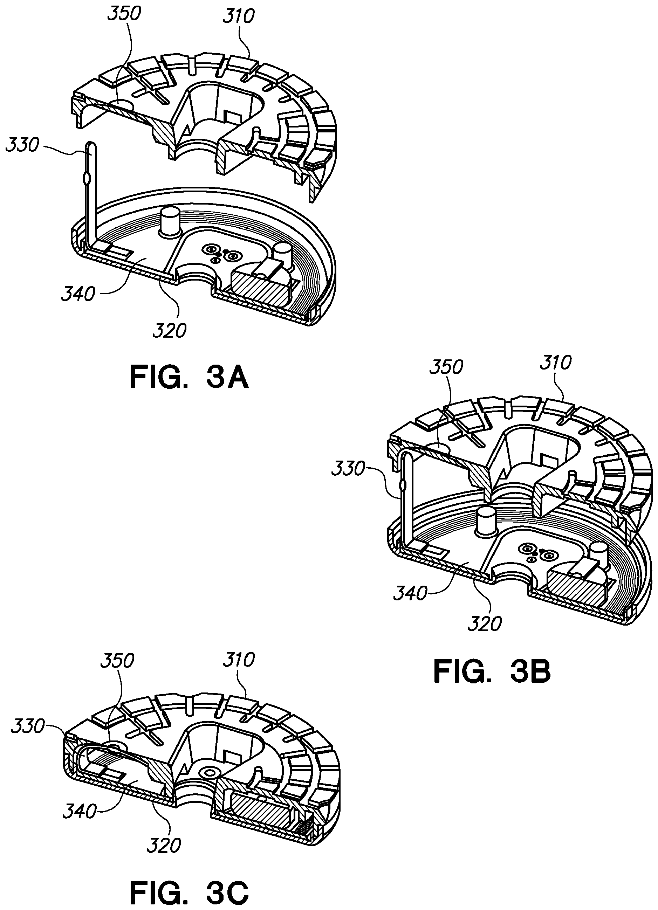

FIGS. 3A-3C illustrate cross sectional perspective views of the thermistor assembly for on body electronics 110 of FIG. 1 in certain embodiments. Referring to FIG. 3A, in certain embodiments, on body electronics 110 of FIG. 1 includes a first housing portion 310 and a second housing portion 320 which are configured to engage each other to form an integrated housing of the on body electronics 110. As shown in FIG. 3A, on the inner surface 340 of the second housing portion 320 is provided a thermistor 330 which is bent upon engaging or assembling the first housing portion 310 and the second housing portion 320 together to form the integrated housing of the on body electronics 110 (FIG. 1). As shown in FIGS. 3A to 3C, when the first housing portion 310 is assembled with the second housing portion 320, in certain embodiments, the thermistor 330 contacts the first housing portion 310 and follows an inner contour (not shown) within an inner surface (not shown) of the first housing portion 310 causing the thermistor 330 to bend. FIG. 3C illustrates the thermistor 330 which has been bent or biased against the inner surface of the first housing portion 310 when the first housing portion 310 and the second housing portion are assembled to form the integrated housing assembly of the on body electronics 110 (FIG. 1).

Referring still to FIG. 3A, also shown on the first housing portion 310 is an opening 350 or a window which, in some embodiments, exposes a portion of the thermistor 330 to a skin surface when the first housing portion 310 is positioned in contact with a skin surface of a user.

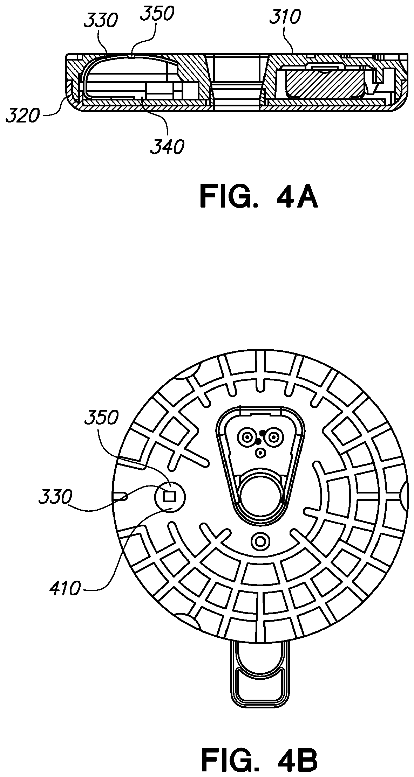

FIGS. 4A and 4B are side cross sectional view and bottom planar views respectively, of the on body electronics 110 with thermistor mounted in certain embodiments. Referring to FIG. 4A, the cross sectional view of the on body electronics 110 (formed of the first and second housing portions 310, 320) illustrates the thermistor 330 having a passively biased portion against the inner surface of the first housing portion 310, while exposing a portion of the thermistor 330 at the opening 350 of the first housing portion 310. As shown in FIG. 4B, in certain embodiments, an adhesive layer 410, such as an ultraviolet (UV) adhesive layer is applied at or around the opening 350 to provide a seal thereof. The adhesive layer 410, in certain embodiments, provides thermal conduction to the thermistor 330, for example, when in direct contact with the skin surface of a user when the on body electronics is positioned on the skin surface.

In the manner described and shown in the corresponding figures, in some embodiments, the configuration of the thermistor 330 provides a simple assembly with the on body electronics 110 (FIG. 1) with accurate positioning and location within the on body electronics 110. In certain embodiments, the thermistor 330 may be pre-biased or pre-bent prior to assembly of the first housing portion 310 and the second housing portion 320, such that the bent shape of the thermistor 330 is retained, while having a portion of the thermistor 330 exposed or otherwise access thereto provided through the opening 350 on the first housing portion 310 of the on body electronics 110 (FIG. 1). Furthermore, while UV adhesive layer is described above as applied over or in the opening 350, any other suitable adhesive layer may be applied to provide a water resistant, or water tight seal of the opening 350 while providing thermal conduction to the thermistor 330.

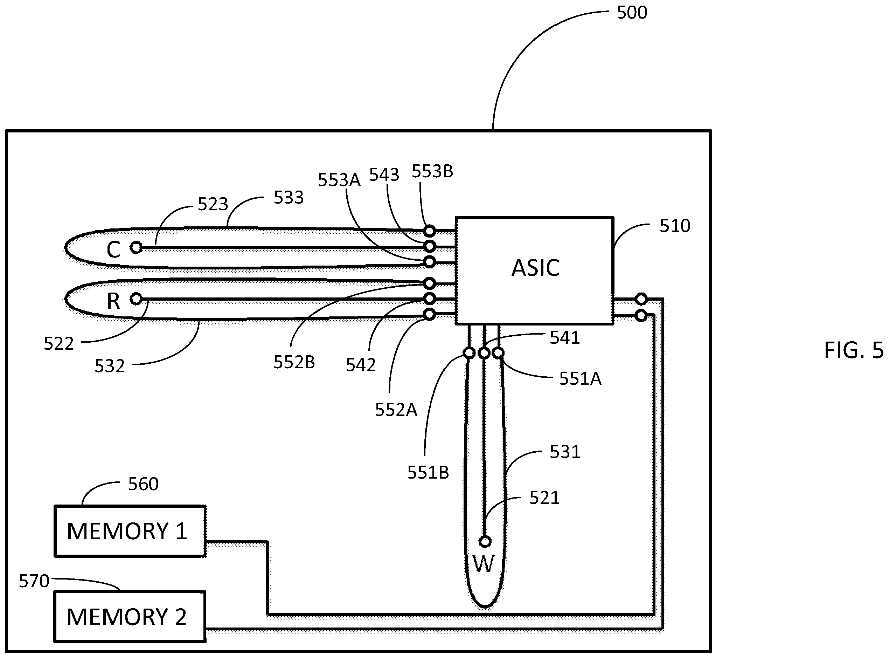

Referring to FIG. 5, in certain embodiments, on body electronics 110 (FIG. 1) includes a printed circuit board (PCB) 500, which, along with a proximal portion of the analyte sensor 101 (FIG. 1) may be encapsulated either partially or entirely, with potting material. Encapsulation of PCB 500 and the proximal portion of the analyte sensor 101 provide protection of the electronic components on the on body electronics 110 provided on PCB 500 from contaminants and/or moisture. In certain embodiments, PCB 500 includes a data processing or control unit such as one or more microprocessors and/or ASICs, one or more memory or data storage devices such as random access memory (RAM), read only memory (ROM), ferromagnetic random access memory (FRAM), electrically erasable programmable read only memory (EEPROM), and the like, to store data and programming and/or control logic or routines to perform the operations related to the processing of signals received from analyte sensor 101. Data processing or control unit may be programmed to perform signal processing such as, for example, but not limited to, current to frequency conversion of the raw sensor signals, analog to digital conversion, signal filtering, storage, data transmission and reception.

Referring to FIG. 5, in some embodiments, on body electronics 110 (FIG. 1) includes an application specific integrated circuit (ASIC) 510 to execute logic for processing signals generated by the analyte sensor 101. In some embodiments, each of the working electrode, reference electrode and counter electrode of the analyte sensor 101 (FIG. 1) is electrically coupled respectively to one of the pins of the ASIC 510. More specifically, as shown in FIG. 5, working electrode 521 of the analyte sensor 101 is connected to the ASIC 510 at the pin 541 and is surrounded by guard trace 531 which are connected to the pins 551A, 551B that are on either sides of the pin 541 that connects the working electrode 521 to the ASIC 510. Similarly, reference electrode 522 of the analyte sensor 101 (FIG. 1) is connected to ASIC 510 at pin 542 and is surrounded by guard trace 532 which are connected to the pins 552A, 552B that are on either sides of the pin 542 that connects the reference electrode 522 to the ASIC 510. Finally, as further shown in FIG. 5, in some embodiments, counter electrode 523 of the analyte sensor 101 is connected to the ASIC 510 at the pin 543 and is surrounded by guard trace 533 which are connected to the pins 553A, 553B that are on either sides of the pin 543 that connects the counter electrode 523 to the ASIC 510. In some embodiments, guard traces 531, 532, 533 are copper or other suitable electrical traces provided on the surface of the PCB 500 and connected to the respective pins to the ASIC 510.

Referring still to FIG. 5, in certain embodiments, ASIC 510 includes an amplifier such as for example, but not limited to, a unity gain buffer amplifier that is configured to drive the reference guard trace 532 and the counter guard trace 533 at substantially the same voltage as the reference electrode 522 and at the counter electrode 523, respectively, of the analyte sensor 101 (FIG. 1). In this manner, in certain embodiments, the signals at the electrodes of the sensor 101 are protected from any signal degradation source such as, for example, surface contamination or moisture that may potentially degrade the signals at the analyte sensor electrodes, resulting in erroneous measurements. In certain embodiments, the unity gain buffer amplifier of the ASIC 510 is configured to maintain a voltage different between the reference electrode 522 and reference guard trace 532 at less than about 200µV. Similarly, in certain embodiments, the unity gain buffer amplifier of the ASIC 510 is configured to maintain a voltage different between the counter electrode 523 and counter guard trace 533 at less than about 200µV.

In certain embodiments, ASIC 510 may also provide additional functions, such as data encryption, data compression, providing or communicating a serial number, time stamp and temperature readings, operating logic, and other functions, in addition to digitizing and transmitting data packets and/or signals corresponding to measured analyte levels. Furthermore, on body electronics 110 (FIG. 1) in certain embodiments include one or more memory devices 560, 570 to store, write, update data, executable algorithm and the like. In certain embodiment, ASIC 510 includes storage devices such as memory devices for storing and updating data and/or storing executable algorithms. In certain embodiments, memory devices may include electrically erasable programmable read only memory (EEPROM), erasable programmable read only memory (EPROM), random access memory (RAM), read only memory (ROM), flash memory, ferromagnetic random access memory (FRAM), or one or more combinations thereof.

In certain embodiments, ASIC 510 includes on chip a RISC (reduced instruction set computing) processor, an EEPROM, and a register (A/D converter operatively coupled to an analyte sensor). EEPROM in certain embodiments includes a portion that has programmed in it one or more characteristics or details associated with a memory management routine. Exemplary characteristics or details include, for example, a source address (e.g., whether it is an array or a single memory location), a destination address, a size/number of bytes to copy to memory, whether the memory location is a loop buffer (e.g., overwriting the older stored values with new values when the end of the buffer is reached).

In certain embodiments, a preset number of specific events may be defined and stored. For example, such events may include, but not limited to, (1) RF power on event, (2) RF data read command, (3) RF data log command, (4) 1 minute data ready event (e.g., the A/D conversion of the signal from the analyte sensor is complete and the digitized data is ready for storage), or (5) log data (10 minute analyte data) ready event (e.g., when 10 minutes of analyte data is available for storage). For example, 10 minutes of analyte data is available in certain embodiments when the last A/D conversion for the 10 minute analyte data is complete. In certain embodiments, other events or states may be defined.

In certain embodiments, when the RISC processor detects one of the specific events, the RISC processor executes the programmed memory management routine. During the execution of the memory management routine, the stored characteristics in EEPROM are retrieved. Based on the retrieved characteristics, the memory management routine stores data associated with the detected event. For example, in certain embodiments, when a RF data log command event is detected, the data associated with this event is logged in another section of the EEPROM on ASIC chip in accordance with the retrieved characteristics (e.g., source and destination address for the data associated with this event).

In certain embodiments, the characteristics stored in EEPROM associated with the specific events may be modified. For example, the source and destination address may be changed or modified to point to a different memory device or storage unit of on body electronics 110 (e.g., a separate EEPROM or memory that is not part of the ASIC chip). For example, data logger applications of the monitoring system 100 requires storing an amount of data (e.g., data for about 30 days, about 45 days, about 60 days or more, of 1 minute interval sampled analyte data (or 5 minute interval sampled data, or 10 minute interval sampled data)) in on body electronics 110 much greater than in on demand application where a limited amount of data is stored (e.g., 15 samples of 1 minute interval sampled analyte data, and 6 hours of historical 10 minute interval sampled analyte data). In certain embodiment, the amount of data for storage in data logger application may exceed the capacity of on chip EEPROM. In such cases, a larger capacity, off chip EEPROM may be provided in on body electronics 110 for storing data from the data logger application. To configure on body electronics 110 to store sampled analyte data in the larger capacity, off chip EEPROM, in certain embodiments, the characteristics stored in EEPROM associated with the events are reprogrammed or updated (for example, by updating the source and destination addresses associated with the events) so that data logging or storage is pointed to the larger off chip EEPROM.

In this manner, by updating or reprogramming the portion of on chip EEPROM that stores the event characteristics, location of data storage in on body electronics 110 may be updated or modified depending upon the desired application or use of on body electronics 110. Furthermore, other stored characteristics associated with one or more particular events may be updated or reprogrammed in EEPROM as desired to modify the use or application of on body electronics 110 in analyte monitoring system 100. This is further advantageously achieved without reprogramming or modifying the stored routines for executing the particular events by the RISC processor.

Prior to initialization of the on body electronics 110 (FIG. 1) for use, there may be a period of time post manufacturing during which on body electronics 110 may be placed in sleep or idle mode. To initialize on body electronics 110 to transition from the sleep or idle mode, in certain embodiments, wireless signal may be provided to on body electronics 110 which, upon receipt by on body electronics 110 initiates an initialization routine to turn on body electronics 110 into operational mode, for example, by turning on its power source.

In certain embodiments, during the sleep or idle mode, the power supply or the battery in the on body electronics 110 is disconnected so that it is not drained prior to actual use, and can support the desired use time period (e.g., 30 days, 20 days, 14 days, 7 days, etc). To prevent inadvertent activation of the on body electronics 110 (for example, by the presence of RF energy source nearby which accidentally activates the on body electronics 110 prior to actual intended use), on body electronics 110 in certain embodiments performs a sensor detection/activation routine to confirm the active use state of the analyte sensor for the intended monitoring.

More specifically, in certain embodiments, upon activation of the battery, on body electronics 110 transitions out of the sleep or idle mode, and is configured to initiate sensor data collection routine to collect and process a predetermined number of sensor data based on the current signals from the analyte sensor 101. The predetermined number of sensor data (for example, 10 minutes, 8 minutes, 5 minutes and so on), are then averaged in one embodiment, and the averaged signal level is compared against a predetermined threshold level which is programmed and associated with the activation status of the on body electronics 110. If it is determined that the averaged sensor signal level is greater than the predetermined threshold level, then the activation status of the on body electronics 110 is declared or determined to be valid, and the programmed operation of the on body electronics 110 continues in one embodiment. On the other hand, if the determined average sensor signal level is less than the predetermined threshold level, then the activation status is determined to be invalid. When the activation status is determined to be invalid, the on body electronics 110 in certain embodiments is configured to turn off the power supply (e.g., battery) using, for example, an electronic switch, and return to the idle sleep state, drawing little or no power from the power supply. In certain embodiments, the predetermined threshold level is determined based on a signal level considered to be valid based upon multiple sensor sampling during manufacturing. Once returned to idle or sleep state, the on body electronics 110 can again be activated using an activation signal from, for example, display device 120. Upon activation, the on body electronics 110 performs the sensor detection/activation routine as described above.

In certain embodiments, when the sensor detection/activation routine has failed, based on the routine described above, a preset number of times, on body electronics 110 may be programmed to enter a fail state so that it is no longer activatable or usable. For example, if an on body electronics 110 has executed five failed sensor detection/activation routines, the on body electronics 110 enters the fail state such that it is not responsive to any activation signal and is not usable.

In some embodiments, sensor detection/activation routine includes the comparison of the initial current signal level excursion when the on body electronics is first powered on. More specifically, in certain embodiments, in the initial turn-on phase, the sensor generates a higher than normal signal level which can be measured and used to detect the presence of an active sensor. For example, during the first 5 or 10 second time interval (or some other suitable time interval such as 30 seconds or 60 seconds) at the start of the on body electronics 110 power on state, the on body electronics 110 monitors the signal level from the sensor, and when an expected signal spike followed by signal settling is detected from the sensor during the initial time interval, the presence of an active sensor is confirmed and the on body electronics 110 may be configured to initiate the programmed data processing cycles. As the presence of the initial signal spike and settling corresponds to the signal characteristics from an analyte sensor in interstitial fluid when poise voltage is applied to the sensor, such initial signal spike followed by signal settling may be detected and used to confirm the presence of the analyte sensor and confirm the activation of the on body electronics 110.

After pairing is complete, when display device 120 queries on body electronics 110 for real time monitored analyte information and/or logged or stored analyte data, in certain embodiments, the responsive data packet transmitted to display device 120 includes a total of 418 bytes that includes 34 bytes of status information, time information and calibration data, 96 bytes of the most recent 16 one-minute glucose data points, and 288 bytes of the most recent 15 minute interval glucose data over the 12 hour period. Depending upon the size or capacity of the memory or storage unit of on body electronics 110, data stored and subsequently provided to the display device 120 may have a different time resolution and/or span a longer or shorter time period. For example, with a larger data buffer, glucose related data provided to the display device 120 may include glucose data over a 24 hour time period at 15 minute sampling intervals, 10 minute sampling intervals, 5 minute sampling intervals, or one minute sampling intervals. Further, the determined variation in the monitored analyte level illustrating historical trend of the monitored analyte level may be processed and/or determined by the on body electronics 110, or alternatively or in addition to, the stored data may be provided to the display device 120 which may then determine the trend information of the monitored analyte level based on the received data packets.

Furthermore, other data types and indicator flags may be stored in the data packet that is communicated to the display device 120 upon request or query from the display device 120. For example, in certain embodiments, the sensor calibration status and/or sensor sensitivity may be stored in the memory unit or data storage unit of the on body electronics 110 and one or more corresponding data identifier may be retrieved and provided to the data packet that is generated and communicated with the glucose data. In certain embodiments, other data, identifier or associated information may be stored in the memory unit of the on body electronics during manufacturing and retrieval, when appropriate or prompted (based on memory location, for example), included in the data packet that is generated by the on body electronics 110 for communication to the data requesting device such as the display device 120. For example, in certain embodiments, during manufacturing, data or information specific to the use of the on body electronics 110 may be stored or programmed into its memory. Such information may include geographical location information where the on body electronics 110 is going to be used or activated, sensor sensitivity information, sensor expiration information, sensor use duration information (30 days, 15 days, 14, days, 13, days, 12 day, 7 days, etc), sensor manufacturing date, sensor activation threshold level, and/or other device diagnostic information which in some embodiments, are used to perform pre-processing during or after the initial activation of the on body electronics 110.

The size of the data packets provided to display device 120 from on body electronics 110 may also vary depending upon the communication protocol and/or the underlying data transmission frequency-whether using a 433 MHz, a 13.56 MHz, or 2.45GHz in addition to other parameters such as, for example, the availability of data processing devices such as a microprocessor (e.g., central processing unit CPU) in on body electronics 110, in addition to the ASIC state machine, size of the data buffer and/or memory, and the like.

Referring back to FIG. 1, in certain embodiments, analyte monitoring system 100 may store the historical analyte data along with a date and/or time stamp and/or contemporaneous temperature measurement, in memory, such as a memory configured as a data logger as described above. In certain embodiments, analyte data is stored at the frequency of about once per minute, or about once every ten minutes, or about once an hour, etc. Data logger embodiments may store historical analyte data for a predetermined period of time, e.g., a duration specified by a physician, for example, e.g., about 1 day to about 1 month or more, e.g., about 3 days or more, e.g., about 5 days or more, e.g., about 7 days or more, e.g., about 2 weeks or more, e.g., about 1 month or more.

Other durations of time may be suitable, depending on the clinical significance of the data being observed. The analyte monitoring system 100 may display the analyte readings to the subject during the monitoring period. In some embodiments, no data is displayed to the subject. Optionally, the data logger can transmit the historical analyte data to a receiving device disposed adjacent, e.g., in close proximity to the data logger. For example, a receiving device may be configured to communicate with the data logger using a transmission protocol operative at low power over distances of a fraction of an inch to about several feet. For example, and without limitation, such close proximity protocols include Certified Wireless USB™, TransferJet™, Bluetooth® (IEEE 802.15.1), WiFi™ (IEEE 802.11), ZigBee® (IEEE 802.15.4-2006), Wibree™, or the like.

In certain embodiments, on body electronics 110 is configured to process the signals obtained from the sensor based on a predetermined processing cycle. For example, in some embodiments, ASIC 510 of the on body electronics 110 is configured to define a data processing cycle of, for example, 15 minutes, 10 minutes, 12 minutes, 5 minutes, 2 minutes, 1 minute or 30 seconds or some other suitable time interval, during which specific programmed data processing routines are executed. In certain embodiments, during the defined data processing cycle, ASIC 510 is configured to enter an active data processing mode and perform one or more data collection routines based on the sensor signals (for example, raw current signals generated by the sensor 101). More specifically, in certain embodiments, during each data processing cycle, ASIC 510 is configured to execute programming logic to, for example, sequentially process a set number of raw sensor current signals as well as other monitored or associated data such as, for example, monitored temperature. In some embodiments, for data processing cycle, ASIC 510 is configured to process a series of defined sensor signals, followed by the on skin temperature, the PCB board temperature, the current signal level at the counter electrode of the analyte sensor 101 (FIG. 1), and any other programmed signal processing to be performed.

For example, in some embodiments, ASIC 510 of the on body electronics 110 is configured to perform 10 seconds of data collection five times sequentially at the beginning of each 15 minute data processing cycle, followed by processing or determining the on skin temperature (for example, from thermistor 330), the PCB or board temperature, and/or the counter electrode current signal level. Each of the five measured or processed sensor signal, temperature measurements and counter electrode current signal are processed to generate the data packet corresponding to the 15 minute data processing cycle. The generated data packet may be stored in one or more of the memory devices, and/or communicated to a remote location such as the display device 120 upon receiving a request for and in response to the request for the generated data packet from the display device 120. When the on body electronics 110 completes the data processing to generate the data packet as described above during the data processing cycle, the on body electronics 110 in certain embodiments returns to an idle state that draws or uses less power from the power source such as a battery within the on body electronics 110 until the next data processing cycle begins.

In certain embodiments, when the request for the real time glucose data is received from the display device 120 during the time that the on body electronics 110 is in active data processing cycle to generate the data packet, one or more of the data collection routines may be disrupted by the RF energy received from the display device 120 that may interfere with the data collection routines. For example, if the real time glucose data request is received during the second of the five 10 second data collection routines for sensor data collection and processing within the 15 minute data processing cycle, the second 10 second data collection routine may be disrupted by the RF energy received from the display device 120. When on body electronics 110 detects such disruption (i.e., exposure to the RF energy during active data processing cycle), in certain embodiments, the on body electronics 110 is configured to perform another data collection routine to replace the disrupted or otherwise degraded data resulting from the interrupted data collection routine.

By way of an example, on body electronics 110 in some embodiments, may perform another 10 second data collection routine either immediately upon detection of the RF energy, or upon completion of the remaining data processing routines (e.g., the remaining three 10 second sensor data processing, temperature data processing, and counter electrode signal determination) during the same 15 minute data processing cycle and generate another sensor data. The resulting additional sensor data from the additional sensor data collection routine is then used to generate the data packet for subsequent storage in memory or communication to the display device 120.

In still other embodiments, when the on body electronics 110 detects the presence of signal interference such as the RF energy from the display device 120 during its active data processing cycle, the on body electronics 110 may be configured to discontinue the data processing cycle in process, and restart the data processing cycle by initiating the first of the five 10 second sensor data collection routines, for example.

As discussed above, in certain embodiments, the sensor data collection may be programmed as a 10 second sensor data collection period, 8 second sensor data collection period, 5 second sensor data collection period, 3 second sensor data collection period or some other suitable time period for collecting multiple sensor data to generate the data packet. Furthermore in certain embodiments, five separate sensor data collection routines are performed to generate the data packet, while in certain other embodiments, four separate sensor data collection routines may be performed, or three separate sensor data collection routines may be performed, or any other suitable number of separate sensor data collection routines may be performed during each active data processing cycle of the on body electronics 110 for generating the data packet representative of the real time glucose data monitored by the analyte sensor 101.

In certain embodiments, when the active data processing cycle of the on body electronics is relatively short (for example one minute), and the interaction with the data requesting device such as the display device 120 is relatively frequent (multiple times during a one minute time interval, for example), there may be instances where the RF energy from the display device 120 may disrupt the data collection routines in the on body electronics 110 during its active data processing cycle. In such instance, in certain embodiments, the user operating the display device 120 may be limited to a predetermined time interval between each data request (that results in exposing the RF energy to the on body electronics 110 potentially during one or more of the active data processing cycle). For example, the user may be limited one data request for each 5 minute interval, or a four minute interval, or a three minute interval or a two minute interval, such that the display device 120 may be configured to disable the activation of the switch or button to request the data on the display device 120 to initiate data transfer from the on body electronics 110.

In certain embodiments, on body electronics 110 is configured to manage the storing or logging of information associated with detected error resulting from the operation of the on body electronics 110 and/or signals from the analyte sensor. For example, given particular memory or storage unit capacity in the on body electronics 110, where certain memory locations are dedicated or assigned for certain types of data, on body electronics 110 may be configured to dynamically manage error logging based on the detected error and the corresponding data bits needed to store the error data bits.

More specifically, in certain embodiments, a preset number of memory locations (for example, 3 or 5 or 7 memory locations or other suitable number of memory locations) are made available for error bits for each glucose sample data. When the detected error results in actual error memory allocation greater than the number preset or pre-assigned, in certain embodiments, the least significant bits for the memory location assigned to store the on body electronics circuit board temperature data may be used to store the error bits that exceed the preset number of error bits. For example, in the case where the preset number of error memory locations is five, and the actual error memory allocation needed is seven for a given glucose sample, on body electronics 110 may be configured to store error bits in the 4 out of the 5 error memory locations in the data storage device, and reserve the 5th error memory location as an identifier bit that indicates whether additional memory locations are used for the additional error bits for storage (for example, the remaining 3 of the 7 error bits that are stored in the least significant bits (LSBs) in the memory location allocated for the circuit board temperature data).

Accordingly, in certain embodiments, for each glucose data packet generated corresponding to the sampled glucose level, on body electronics may be configured to dynamically allocate LSBs of the memory locations assigned or set aside for circuit board temperature data to store or log extra or additional error data bits that are not stored in the preset error memory locations in the data storage device. In this manner, all error data bit generated that correspond to each glucose sample is stored in the memory structure of the data storage device or memory unit of the on body electronics 110, by, for example, reallocating some LSBs of the memory location dedicated for non-error data type, without sacrificing the accuracy of the other data type (for example, the board temperature data in the example provided above), as only the LSBs are used for storing error bits.

Various other modifications and alterations in the structure and method of operation of this disclosure will be apparent to those skilled in the art without departing from the scope of the embodiments of the present disclosure. Although the present disclosure has been described in connection with particular embodiments, it should be understood that the present disclosure as claimed should not be unduly limited to such particular embodiments. It is intended that the following claims define the scope of the present disclosure and that structures and methods within the scope of these claims and their equivalents be covered thereby.

## Claims

1. An apparatus (100), comprising:
a glucose sensor (101, 110) including a working electrode (521) having a portion (103) in fluid contact with interstitial fluid under a skin surface;
a memory unit (560, 570) having a first plurality of memory locations configured to store a first predetermined type of data and a second plurality of memory locations configured to store a second predetermined type of data; and
a processing unit (160, 510) operatively coupled to the glucose sensor (101, 110) and the memory unit (560, 570), the processing unit (160, 510) configured to process analyte related signals generated by the glucose sensor (101, 110), and to control data storage in and retrieval from the memory unit (560, 570);
wherein, the first predetermined type of data includes error data, the second predetermined type of data includes monitored physiological condition data, wherein when an error condition associated with operation of the glucose sensor (101, 110) is detected by the processing unit (160, 510), the processing unit is configured to store data associated with the first predetermined type of data in a subset of the second plurality of memory locations of the memory unit (560, 570); and further
wherein, the processing unit (160, 510) is configured to store an error condition indicator in a subset of the first plurality of memory locations of the memory unit (560, 570), and
**characterised in that**, when the error condition is detected by the processing unit (160, 510), a first portion of a plurality of error data bits corresponding to the detected error condition is stored in the subset of the first plurality of memory locations, and a second portion of the plurality of error data bits is stored in the subset of the second plurality of memory locations, the first portion and the second portion comprising the entire error data bits.

2. The apparatus (100) of claim 1, wherein the first predetermined type of data includes the error data corresponding to the error condition associated with the operation of the glucose sensor (101, 110).

3. The apparatus (100) of claim 1 or claim 2, wherein the monitored physiological condition data corresponding to the second predetermined type of data includes one or more of temperature data, analyte level data, pressure data, humidity data, or sterility data.

4. The apparatus (100) of any preceding claim, further comprising a data communication module operatively coupled to the processing unit (160, 510), the data communication module configured to communicate data stored in the first plurality of memory locations and the second plurality of memory locations in a single data packet to a remote location (120, 170), preferably wherein the data communication module wirelessly communicates the single data packet to the remote location in response to a request for data received from the remote location.

5. The apparatus (100) of any preceding claim, wherein the first plurality of memory locations of the memory unit (560, 570) has storage capacity to store only the first portion of a plurality of error data bits and the error condition indicator and/or, wherein the second plurality of memory locations of the memory unit has storage capacity to store only the second portion of a plurality of error data bits and a subset of the second predetermined type of data including the monitored physiological condition data, preferably wherein the subset of the second predetermined type of data stored in the second plurality of memory locations has sufficient bit resolution to represent the monitored physiological condition data.

6. The apparatus (100) of any preceding claim, wherein the second portion of a plurality of error data bits are stored in the least significant bit locations in the second plurality of memory locations.

7. The apparatus (100) of any preceding claim, wherein the memory unit (560, 570) further includes a third plurality of memory locations for storing a third predetermined type of data including data associated with calibration status identifier of the glucose sensor (101, 110), preferably wherein the processing unit updates the stored calibration status identifier based on a signal condition received from the glucose sensor.

8. The apparatus (100) of any preceding claim, wherein the memory unit (560, 570) further includes a third plurality of memory locations for storing a third predetermined type of data including data associated with glucose sensor usage duration, preferably wherein the data associated with the glucose sensor usage duration includes sensor expiration data.

9. The apparatus (100) of any preceding claim, wherein the memory unit (560, 570) further includes a third plurality of memory locations for storing a third predetermined type of data including data associated with glucose sensor geographical usage location.

10. The apparatus (100) of any preceding claim, wherein the memory unit (560, 570) further includes a third plurality of memory locations for storing a third predetermined type of data including data associated with glucose sensor manufacturing information, preferably wherein the data associated with the glucose sensor manufacturing information includes glucose sensor sensitivity data.

11. The apparatus (100) of any preceding claim, wherein the memory unit (560, 570) further includes a third plurality of memory locations for storing a third predetermined type of data including data associated with glucose sensor usage type, preferably wherein the data associated with the glucose sensor usage type includes a retrospective glucose sensor data collection operation, and/or wherein the data associated with the glucose sensor usage type includes real time glucose sensor data acquisition and output operation.

12. A method, comprising:
detecting an error condition related to glucose sensor (101, 110) operation;
generating error data corresponding to the detected error condition;
storing a first portion of the generated error data in a first predetermined memory location (560, 570) configured for storing the error data;
storing a second portion of the generated error data in a second predetermined memory location (560, 570) configured for storing monitored physiological data; and
generating an error data flag and storing the error data flag in the first predetermined memory location configured for storing the error data.

13. The method of claim 12, further comprising:
generating a single data packet including the error data stored in the first and second predetermined memory locations (560, 570); and
wirelessly transmitting to a remote location (120, 170) the single data packet in response to a data request received from the remote location.

14. The method of claim 12 or 13, wherein the first predetermined memory location (560, 570) has a storage capacity to store only the first portion of the generated error data and the generated error data flag.

15. The method of claim 12, 13 or 14, wherein the second predetermined memory location (560, 570) has a storage capacity to store only the second portion of the generated error data and a subset of the monitored physiological data.

## Patentansprüche

1. Vorrichtung (100), die Folgendes umfasst:
einen Glukosesensor (101, 110), der eine Arbeitselektrode (521) umfasst, die einen Abschnitt (103) in Flüssigkeitskontakt mit interstitieller Flüssigkeit unter einer Hautoberfläche aufweist;
eine Speichereinheit (560, 570), die eine erste Vielzahl von Speicherstellen, die zum Speichern eines ersten vorgegebenen Datentyps konfiguriert sind, und eine zweite Vielzahl von Speicherstellen, die zum Speichern eines zweiten vorgegebenen Datentyps konfiguriert sind, aufweist und
eine Verarbeitungseinheit (160, 510), die betriebsmäßig mit dem Glucosesensor (101, 110) und der Speichereinheit (560, 570) gekoppelt ist, wobei die Verarbeitungseinheit (160, 510) dafür konfiguriert ist, analytenbezogene Signale zu verarbeiten, die von dem Glukosesensor (101, 110) erzeugt werden, und die Datenspeicherung in der Speichereinheit (560, 570) und den Abruf von dieser zu steuern;
wobei der erste vorbestimmte Datentyp Fehlerdaten umfasst, der zweite vorbestimmte Datentyp überwachte physiologische Zustandsdaten umfasst, wobei, wenn durch die Verarbeitungseinheit (160, 510) ein Fehlerzustand im Zusammenhang mit dem Betrieb des Glucosesensors (101, 110) erfasst wird, die Verarbeitungseinheit dafür konfiguriert ist, Daten, die dem ersten vorbestimmten Datentyp verbunden sind, in einer Teilmenge der zweiten Vielzahl von Speicherstellen der Speichereinheit (560, 570) zu speichern; und ferner
wobei die Verarbeitungseinheit (160, 510) dafür konfiguriert ist, einen Fehlerzustandsindikator in einer Teilmenge der ersten Vielzahl von Speicherstellen der Speichereinheit (560, 570) zu speichern und
**dadurch gekennzeichnet, dass** wenn der Fehlerzustand durch die Verarbeitungseinheit (160, 510) erfasst wird, ein erster Teil einer Vielzahl von Fehlerdatenbits, die dem erfassten Fehlerzustand entsprechen, in der Teilmenge der ersten Vielzahl von Speicherstellen gespeichert wird und ein zweiter Teil der Vielzahl von Fehlerdatenbits in der Teilmenge der zweiten Vielzahl von Speicherstellen gespeichert wird, wobei der erste Teil und der zweite Teil die gesamten Fehlerdatenbits umfassen.

2. Vorrichtung (100) nach Anspruch 1, wobei der erste vorbestimmte Datentyp die Fehlerdaten umfasst, die dem Fehlerzustand entsprechen, der mit dem Betrieb des Glukosesensors (101, 110) verbunden ist.

3. Vorrichtung (100) nach Anspruch 1 oder Anspruch 2, wobei die überwachten physiologischen Zustandsdaten, die dem zweiten vorbestimmten Datentyp entsprechen, Temperaturdaten, Analytniveaudaten, Druckdaten, Feuchtigkeitsdaten oder Sterilitätsdaten umfassen.

4. Vorrichtung (100) nach einem der vorhergehenden Ansprüche, die ferner ein Datenkommunikationsmodul umfasst, das betriebsmäßig mit der Verarbeitungseinheit (160, 510) gekoppelt ist, wobei das Datenkommunikationsmodul dafür konfiguriert ist, Daten, die in der ersten Vielzahl von Speicherstellen und der zweiten Vielzahl von Speicherstellen in einem einzelnen Datenpaket gespeichert sind, an einen entfernten Ort (120, 170) zu übertragen, wobei das Datenkommunikationsmodul das einzelne Datenpaket vorzugsweise als Antwort auf eine Anfrage nach Daten, die von dem entfernten Ort empfangen wurden, drahtlos an den entfernten Ort überträgt.

5. Vorrichtung (100) nach einem der vorhergehenden Ansprüche, wobei die erste Vielzahl von Speicherstellen der Speichereinheit (560, 570) eine Speicherkapazität aufweist, um nur den ersten Teil einer Vielzahl von Fehlerdatenbits und den Fehlerzustandsindikator zu speichern/oder, und wobei die zweite Vielzahl von Speicherstellen der Speichereinheit Speicherkapazität aufweist, um nur den zweiten Teil einer Vielzahl von Fehlerdatenbits und eine Teilmenge des zweiten vorbestimmten Datentyps einschließlich der überwachten physiologischen Zustandsdaten zu speichern, wobei die Untermenge des zweiten vorbestimmten Datentyps, die in der zweiten Vielzahl von Speicherstellen gespeichert ist, vorzugsweise eine ausreichende Bitauflösung aufweist, um die überwachten physiologischen Zustandsdaten darzustellen.

6. Vorrichtung (100) nach einem der vorhergehenden Ansprüche, wobei der zweite Teil einer Vielzahl von Fehlerdatenbits an den niedrigstwertigen Bitstellen in der zweiten Vielzahl von Speicherstellen gespeichert ist.

7. Vorrichtung (100) nach einem der vorhergehenden Ansprüche, wobei die Speichereinheit (560, 570) ferner eine dritte Vielzahl von Speicherstellen zum Speichern eines dritten vorbestimmten Datentyps einschließlich Daten, die mit der Kalibrierungsstatuskennung des Glukosesensors (101, 110) verbunden sind, umfasst, wobei die Verarbeitungseinheit vorzugsweise die gespeicherte Kalibrierungsstatuskennung auf der Grundlage eines vom Glukosesensor empfangenen Signalzustands aktualisiert.

8. Vorrichtung (100) nach einem der vorhergehenden Ansprüche, wobei die Speichereinheit (560, 570) ferner eine dritte Vielzahl von Speicherstellen zum Speichern eines dritten vorbestimmten Datentyps einschließlich Daten, die mit der Nutzungsdauer des Glukosesensors verbunden sind, umfasst, wobei die Daten, die mit der Verwendungsdauer des Glukosesensors verbunden sind, vorzugsweise Sensorablaufdaten umfassen.

9. Vorrichtung (100) nach einem der vorhergehenden Ansprüche, wobei die Speichereinheit (560, 570) ferner eine dritte Vielzahl von Speicherstellen zum Speichern eines dritten vorbestimmten Datentyps einschließlich Daten, die mit dem geografischen Verwendungsort des Glukosesensors verbunden sind, umfasst.

10. Vorrichtung (100) nach einem der vorhergehenden Ansprüche, wobei die Speichereinheit (560, 570) ferner eine dritte Vielzahl von Speicherstellen zum Speichern eines dritten vorbestimmten Datentyps einschließlich Daten, die mit den Herstellungsinformationen des Glukosesensors verbunden sind, umfasst, wobei die mit den Herstellungsinformationen des Glukosesensors verbundenen Daten vorzugsweise Glukosesensorsensitivitätsdaten umfassen.

11. Vorrichtung (100) nach einem der vorhergehenden Ansprüche, wobei die Speichereinheit (560, 570) ferner eine dritte Vielzahl von Speicherstellen zum Speichern eines dritten vorbestimmten Datentyps einschließlich Daten, die mit dem Nutzungstyp des Glukosesensors verbunden sind, umfasst, wobei die Daten, die dem Nutzungstyp des Glukosesensors verbunden sind, vorzugsweise eine retrospektive Glukosesensordatensammeloperation umfassen und/oder wobei die Daten, die dem Nutzungstyp des Glukosesensors zugeordnet sind, eine Echtzeit-Glukosesensordatenerfassung und eine Ausgabeoperation umfassen.

12. Verfahren, das Folgendes umfasst:
Erkennen eines Fehlerzustands in Bezug auf den Betrieb des Glukosesensors (101, 110);
Erzeugen von Fehlerdaten entsprechend dem erfassten Fehlerzustand;
Speichern eines ersten Teils der erzeugten Fehlerdaten an einem ersten vorbestimmten Speicherort (560, 570), der zum Speichern der Fehlerdaten konfiguriert ist;
Speichern eines zweiten Teils der erzeugten Fehlerdaten an einem zweiten vorbestimmten Speicherort (560, 570), der zum Speichern von überwachten physiologischen Daten konfiguriert ist; und
Erzeugen eines Fehlerdatenkennzeichens und Speichern des Fehlerdatenkennzeichens an dem ersten vorbestimmten Speicherort, der zum Speichern der Fehlerdaten konfiguriert ist.

13. Verfahren nach Anspruch 12, das ferner Folgendes umfasst:
Erzeugen eines einzelnen Datenpakets, das die in den ersten und zweiten vorbestimmten Speicherstellen (560, 570) gespeicherten Fehlerdaten enthält; und
drahtloses Übertragen des einzelnen Datenpakets an einen entfernten Ort (120, 170) als Antwort auf eine von dem entfernten Ort empfangene Datenanforderung.

14. Verfahren nach Anspruch 12 oder 13, wobei der erste vorbestimmte Speicherort (560, 570) eine Speicherkapazität aufweist, um nur den ersten Teil der erzeugten Fehlerdaten und das erzeugte Fehlerdatenkennzeichen zu speichern.

15. Verfahren nach Anspruch 12, 13 oder 14, wobei der zweite vorbestimmte Speicherort (560, 570) eine Speicherkapazität aufweist, um nur den zweiten Teil der erzeugten Fehlerdaten und eine Teilmenge der überwachten physiologischen Daten zu speichern.

## Revendications

1. Appareil (100), comprenant :
un capteur de glucose (101, 110) incluant une électrode de travail (521) ayant une partie (103) en contact fluidique avec du fluide interstitiel sous une surface cutanée ;
une unité de mémoire (560, 570) ayant une première pluralité d'emplacements de mémoire configurée pour stocker un premier type prédéterminé de données et une deuxième pluralité d'emplacements de mémoire configurée pour stocker un deuxième type prédéterminé de données ; et
une unité de traitement (160, 510) couplée de manière fonctionnelle au capteur de glucose (101, 110) et à l'unité de mémoire (560, 570), l'unité de traitement (160, 510) configurée pour traiter des signaux liés à un analyte générés par le capteur de glucose (101, 110), et pour commander le stockage de données dans l'unité de mémoire (560, 570) et l'extraction de données à partir de celle-ci ;
dans lequel, le premier type prédéterminé de données inclut des données d'erreur, le deuxième type prédéterminé de données inclut des données d'état physiologiques surveillées, dans lequel quand un état d'erreur associé au fonctionnement du capteur de glucose (101, 110) est détecté par l'unité de traitement (160, 510), l'unité de traitement est configurée pour stocker les données associées au premier type prédéterminé de données dans un sous-ensemble de la deuxième pluralité d'emplacements de mémoire de l'unité de mémoire (560, 570) ; et en outre
dans lequel, l'unité de traitement (160, 510) est configurée pour stocker un indicateur d'état d'erreur dans un sous-ensemble de la première pluralité d'emplacements de mémoire de l'unité de mémoire (560, 570), et
**caractérisé en ce que**, quand l'état d'erreur est détecté par l'unité de traitement (160, 510), une première partie d'une pluralité de bits de données d'erreur correspondant à l'état d'erreur détecté est stockée dans le sous-ensemble de la première pluralité d'emplacements de mémoire, et une deuxième partie de la pluralité de bits de données d'erreur est stockée dans le sous-ensemble de la deuxième pluralité d'emplacements de mémoire, la première partie et la deuxième partie comprenant toutes les bits de données d'erreur.

2. Appareil (100) selon la revendication 1, dans lequel le premier type prédéterminé de données inclut les données d'erreur correspondant à l'état d'erreur associé au fonctionnement du capteur de glucose (101, 110).

3. Appareil (100) selon la revendication 1 ou la revendication 2, dans lequel les données d'état physiologiques surveillées correspondant au deuxième type prédéterminé de données incluent une ou plusieurs données parmi des données de température, des données de niveau d'analyte, des données de pression, des données d'humidité, ou des données de stérilité.

4. Appareil (100) selon l'une quelconque des revendications précédentes, comprenant un module de communication de données couplé de manière fonctionnelle à l'unité de traitement (160, 510), le module de communication de données configuré pour communiquer des données stockées dans la première pluralité d'emplacements de mémoire et la deuxième pluralité d'emplacements de mémoire dans un paquet de données unique vers un emplacement éloigné (120, 170), préférablement dans lequel le module de communication de données communique sans fil le paquet de données unique vers l'emplacement éloigné en réponse à une requête de données reçue de l'emplacement éloigné.

5. Appareil (100) selon l'une quelconque des revendications précédentes, dans lequel la première pluralité d'emplacements de mémoire de l'unité de mémoire (560, 570) a une capacité de stockage pour stocker uniquement la première partie d'une pluralité de bits de données d'erreur et l'indicateur d'état d'erreur et/ou, dans lequel la deuxième pluralité d'emplacements de mémoire de l'unité de mémoire a une capacité de stockage pour stocker uniquement la deuxième partie d'une pluralité de bits de données d'erreur et un sous-ensemble du deuxième type prédéterminé de données incluant les données d'état physiologiques surveillées, préférablement dans lequel le sous-ensemble du deuxième type prédéterminé de données stocké dans la deuxième pluralité d'emplacements de mémoire a une résolution de bit suffisante pour représenter les données d'état physiologiques surveillées.

6. Appareil (100) selon l'une quelconque des revendications précédentes, dans lequel la deuxième partie d'une pluralité de bits de données d'erreur est stockée dans les emplacements de bits les moins significatifs dans la deuxième pluralité d'emplacements de mémoire.

7. Appareil (100) selon l'une quelconque des revendications précédentes, dans lequel l'unité de mémoire (560, 570) inclut en outre une troisième pluralité d'emplacements de mémoire destinée à stocker un troisième type prédéterminé de données incluant des données associées à l'identifiant de statut de calibrage du capteur de glucose (101, 110), préférablement dans lequel l'unité de traitement met à jour l'identifiant de statut de calibrage stocké sur la base d'un état de signal reçu du capteur de glucose.

8. Appareil (100) selon l'une quelconque des revendications précédentes, dans lequel l'unité de mémoire (560, 570) inclut en outre une troisième pluralité d'emplacements de mémoire destinée à stocker un troisième type prédéterminé de données incluant des données associées à la durée d'usage du capteur de glucose, préférablement dans lequel les données associées à la durée d'usage du capteur de glucose incluent des données d'expiration du capteur.

9. Appareil (100) selon l'une quelconque des revendications précédentes, dans lequel l'unité de mémoire (560, 570) inclut en outre une troisième pluralité d'emplacements de mémoire destinée à stocker un troisième type prédéterminé de données incluant des données associées à l'emplacement d'usage géographique du capteur de glucose.

10. Appareil (100) selon l'une quelconque des revendications précédentes, dans lequel l'unité de mémoire (560, 570) inclut en outre une troisième pluralité d'emplacements de mémoire destinée à stocker un troisième type prédéterminé de données incluant des données associées à des informations de fabrication du capteur de glucose, préférablement dans lequel les données associées aux informations de fabrication du capteur de glucose incluent des données de sensibilité du capteur de glucose.

11. Appareil (100) selon l'une quelconque des revendications précédentes, dans lequel l'unité de mémoire (560, 570) inclut en outre une troisième pluralité d'emplacements de mémoire destinée à stocker un troisième type prédéterminé de données incluant des données associées au type d'usage du capteur de glucose, préférablement dans lequel les données associées au type d'usage du capteur de glucose incluent une opération de collecte de données rétrospectives du capteur de glucose, et/ou dans lequel les données associées au type d'usage du capteur de glucose incluent l'acquisition de données du capteur de glucose en temps réel et une opération de sortie.

12. Procédé, comprenant :
la détection d'un état d'erreur lié au fonctionnement d'un détecteur de glucose (101, 110) ;
la génération de données d'erreur correspondant à l'état d'erreur détecté ;
le stockage d'une première partie des données d'erreur générées dans un premier emplacement de mémoire prédéterminé (560, 570) configuré pour stocker les données d'erreur ;
le stockage d'une deuxième partie des données d'erreur générées dans un deuxième emplacement de mémoire prédéterminé (560, 570) configuré pour stocker des données physiologiques surveillées ; et
la génération d'un drapeau de données d'erreur et le stockage du drapeau de données d'erreur dans le premier emplacement de mémoire prédéterminé configuré pour stocker les données d'erreur.

13. Procédé selon la revendication 12, comprenant en outre :
la génération d'un paquet de données unique incluant les données d'erreur stockées dans les premier et deuxième emplacements de mémoire prédéterminés (560, 570) ; et
l'émission sans fil vers un emplacement éloigné (120, 170) du paquet de données unique en réponse à une requête de données reçue de l'emplacement éloigné.

14. Procédé selon la revendication 12 ou 13, dans lequel le premier emplacement de mémoire prédéterminé (560, 570) a une capacité de stockage pour stocker uniquement la première partie des données d'erreur générées et le drapeau de données d'erreur généré.

15. Procédé selon la revendication 12, 13 ou 14, dans lequel le deuxième emplacement de mémoire prédéterminé (560, 570) a une capacité de stockage pour stocker uniquement la deuxième partie des données d'erreur générées et un sous-ensemble des données physiologiques surveillées.
